# EUROPEAN PATENT SPECIFICATION

(11) **EP 3 600 090 B1**
(45) Date of publication and mention of the grant of the patent: **15.09.2021**
(21) Application number: 18716058.5
(22) Date of filing: 16.03.2018
(51) Int. Cl.: A61B 17/3207, A61B 17/32

(54) **TISSUE-REMOVING CATHETER WITH ABRASIVE BURR HAVING PORTION FREE FROM ABRASIVE EXTERIOR SURFACE**
GEWEBEENTFERNENDER KATHETER MIT ABRASIVEM FRÄSKOPF DEREN OBERFLÄCHE TEILWEISE NICHT ABRASIV IST
CATHÉTER D'ABLATION DE TISSUS AVEC TÊTE DE FRAISAGE ABRASIVE DONT LA SURFACE EST PARTIELLEMENT NON ABRASIVE

(30) Priority: 20.03.2017 US 201762473546 P
(43) Date of publication of application: 05.02.2020
(73) Proprietor: Covidien LP, Mansfield, MA 02048 (US)
(72) Inventor: STEELE, Bradley, Plymouth, Minnesota 55364 (US); SCHNEIDER, Mark, Mound, Minnesota 55379 (US); MCPEAK, Thomas, Caledonia, Minnesota 55921 (US)
(74) Representative: Zimmermann & Partner Patentanwälte mbB
(86) International application number: PCT/US2018/022843
(87) International publication number: WO 2018/175229

(56) References cited:
- EP-A1- 3 366 239
- US-A- 5 501 694
- US-A1- 2016 374 715

## Description

### FIELD OF THE DISCLOSURE

The present disclosure generally relates to a tissue-removing catheter with a rotatable burr including an abrasive exterior surface, wherein portions of the rotatable burr are free from the abrasive exterior surface.

### BACKGROUND

The patency of a body lumen may be affected by the build-up of tissue or other material in the body lumen. A variety of methods for cutting or dislodging occlusive material and removing such material from a body lumen, such as a blood vessel, have been proposed. For example, tissue-removing catheters may be used to restore the patency of a body lumen. These catheters are intended to cut or excise material from the body lumen and may employ a rotatable tissue-removing element which can be advanced into or past the occlusive material in order to cut and separate such material from the body lumen.

Although these catheters have proven very successful in restoring the patency of body lumens, problems may arise when the tissue-removing element has a smaller diameter than the occlusive tissue. If the tissue-removing element does not maintain contact with the occlusion or lumen wall, the efficacy of the tissue-removing element is reduced.

Document US 2016/0374715 A1 relates to a tissue-removing catheter with adjustable cross-sectional dimension. Document US 5,501,694 relates to an expandable intravascular occlusion material removal devices and methods of use. Document EP 3 366 239 A1 belongs to the state of the art pursuant to Article 54(3) EPC and relates to an intravascular catheter having an expandable incising portion and abrasive surfaces.

### SUMMARY

In one aspect, a tissue-removing catheter generally comprises an abrasive burr including circumferentially spaced longitudinal struts defining longitudinal slots between adjacent struts, proximal and distal hubs secured to the respective proximal and distal ends of the longitudinal struts, and an abrasive exterior surface covering an abrasive longitudinal portion of each strut extending from adjacent the proximal ends of the longitudinal struts to adjacent the distal ends of the longitudinal struts. Proximal and distal longitudinal end portions of the longitudinal struts adjacent the junctions of the longitudinal struts and the respective proximal and distal hubs are free from the abrasive exterior surface. In another aspect, a method of forming an abrasive burr for a catheter is disclosed. The invention is defined by claims 1 and 14. Further embodiments of the invention are defined by the dependent claims.

Other features will be in part apparent and in part pointed out hereinafter.

### BRIEF DESCRIPTION OF THE DRAWINGS

FIG. 1 is a side elevation of an embodiment of a tissue-removing catheter, an abrasive burr of the catheter being in an initial, non-expanded configuration;
FIG. 2 is an enlarged elevational view of the tissue-removing catheter of FIG. 1;
FIG. 3 is an enlarged perspective view of the tissue-removing catheter of FIG. 1;
FIG. 4 is similar to FIG. 2, except with the abrasive burr being in an expanded configuration; and

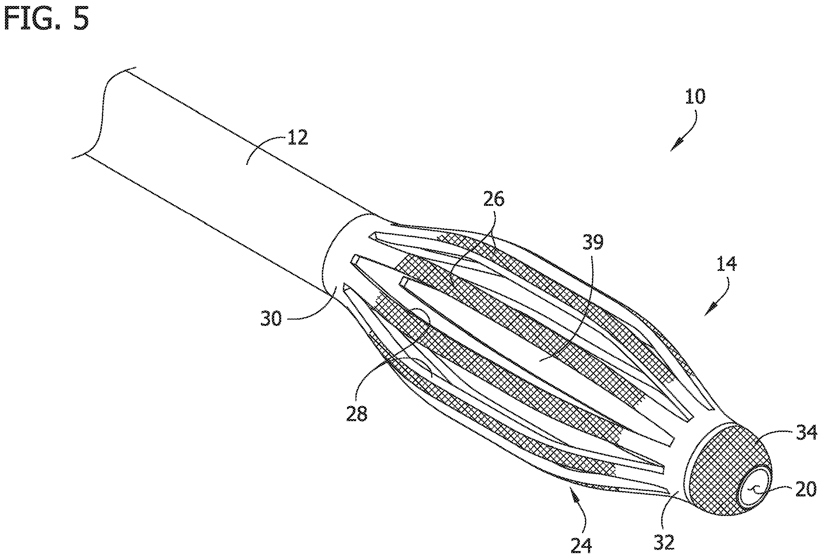

FIG. 5 is similar to FIG. 3, except with the abrasive burr being in the expanded configuration.

Corresponding reference characters indicate corresponding parts throughout the drawings.

### DETAILED DESCRIPTION OF THE DISCLOSURE

Referring to the drawings, an embodiment of a tissue-removing catheter for removing tissue from a body lumen is generally indicated at reference numeral 10. The illustrated tissue-removing catheter 10 is particularly suitable for removing an atheroma (e.g., plaque) from a blood vessel, although the catheter may be used to remove other occlusions from other body lumens.

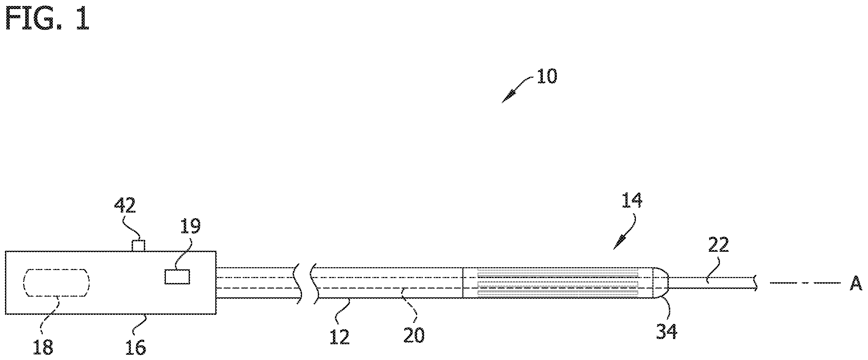

Referring to FIG. 1, the tissue-removing catheter 10 comprises a drive shaft 12, and an abrasive burr, generally indicated at 14, secured to a distal end of the drive shaft and configured to remove tissue (e.g., plaque) from a body lumen (e.g., a blood vessel). A handle 16 is operatively connected to a proximal end portion of the drive shaft 12. A motor 18 (e.g., an electric motor) or other prime mover in or otherwise operatively connected to the handle 16 is configured to rotate the drive shaft 12 and the abrasive burr 14 about an axis A (e.g., longitudinal axis of the catheter 12) to remove tissue from a lesion or other obstruction in a body lumen. The handle 16 may include a button or lever or other actuator 19 that is operatively connected to the motor 18 for actuating rotation of the drive shaft 12 and the burr 14. A guide wire lumen 20 extends longitudinally through the catheter body 12 and the abrasive burr 14 to receive a guide wire 22, which may extend distally outward from the distal end of the burr. In this way, the illustrated tissue-removing catheter 10 is configured for use as an over-wire device (i.e., over the guide wire 22). A specialty wire may be used in conjunction with the tissue-removing catheter 10 to facilitate coring through total or near total occlusions, for example. Other configurations are within the scope of the present invention, and it is understood that the guide wire 22 may be omitted within the scope of the present invention.

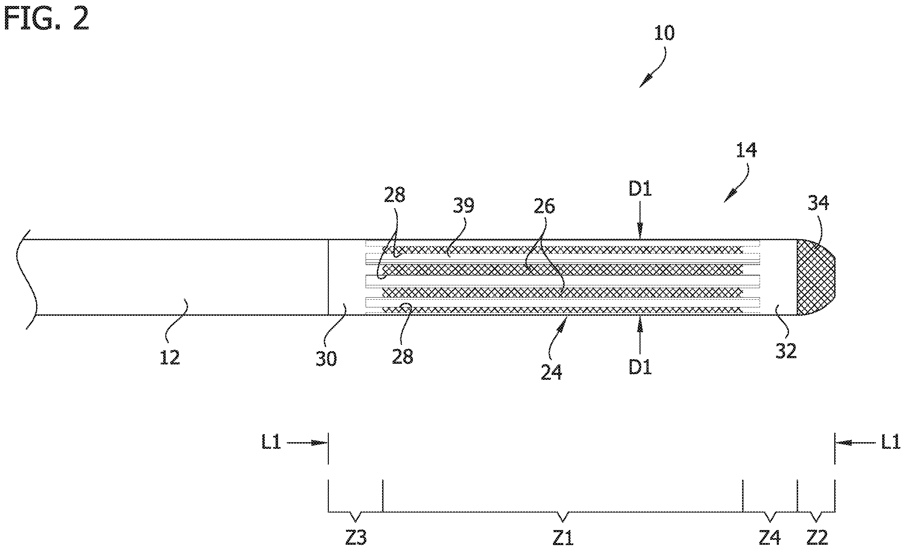

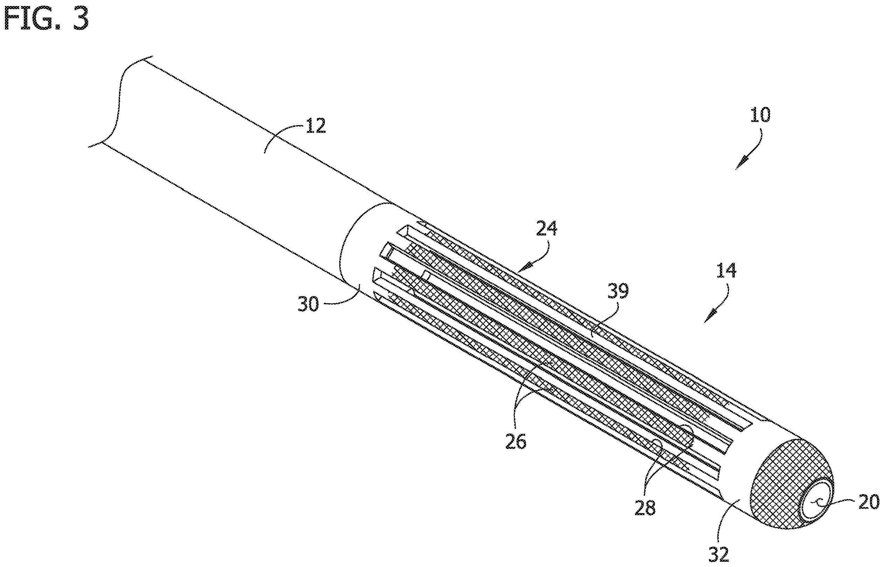

Referring to FIGS. 2 and 3, the abrasive burr 14 includes a generally tubular body, generally indicated at 24, including circumferentially spaced longitudinal struts 26 (or fingers) defining longitudinal slots 28 between adjacent struts, and proximal and distal hubs (e.g., annular hubs) 30, 32, respectively, secured to the respective proximal and distal ends of the struts. The longitudinal slots 28 permit the struts 26 to expand circumferentially relative to the proximal and distal hubs 30, 32, respectively, as described below. The abrasive burr 14 further includes a distal head 34 having a generally conical or dome-shape that tapers distally. The distal head 34 may be suitable for boring through tissue (e.g., plaque) occluding a body lumen. The tubular body 24 and the distal head 34 may be a single, one-piece component that is integrally, monolithically formed from a single piece of material. For example, the tubular body 24 and the distal head 34 may be formed from a single piece of hypotube. In other examples, the tubular body 24 and the distal head 34 may be formed separately and secured to one another in any suitable way. The tubular body 24 and the distal head 34 may be made of Nitinol, spring steel, stainless steel, or any other suitable material.

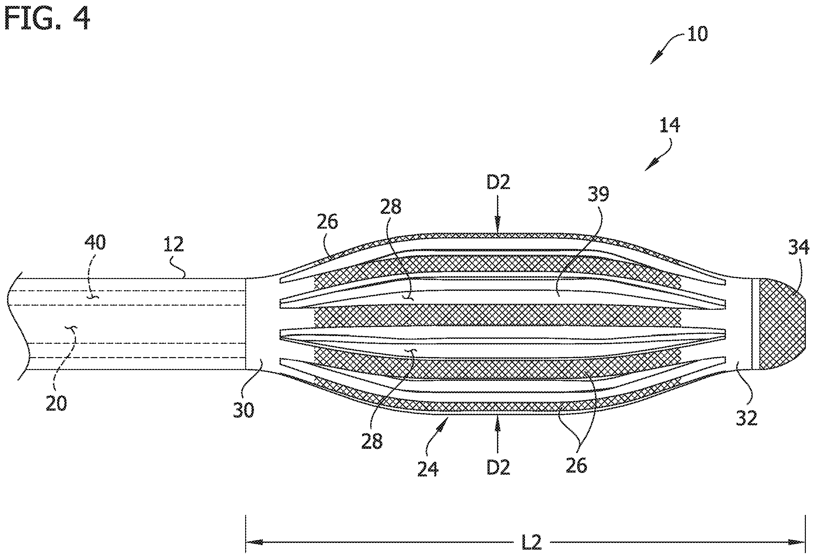

The abrasive burr 14 has an initial, non-expanded or minimum cross-sectional dimension D1 (see FIG. 2) and a first longitudinal length L1. The abrasive burr 14 is expandable circumferentially to increase the cross-sectional dimension to an expanded or maximum cross-sectional dimension D2 (FIG. 4), which is larger than the initial cross-sectional dimension D1. As illustrated, the initial cross-sectional dimension D1 is about the same as a cross-sectional dimension of the drive shaft 12, and the expanded cross-sectional dimension D2 is larger than the cross-sectional dimension of the drive shaft, although other configurations are within the scope of the present invention. In the expanded configuration, the struts 26 of the abrasive burr 14 flex or bend (broadly, deflect) outward to increase the cross-sectional dimension of the burr, which, in turn, shortens the length L1 of the burr to a second longitudinal length L2 (FIG. 4). Thus, as the cross-sectional dimension of the abrasive burr 14 increases, the longitudinal length of the abrasive burr decreases. In some embodiments, the abrasive burr 14 can be expanded and contracted to have any cross-sectional dimension in the range between the initial cross-sectional dimension D1 and a maximum expanded cross-sectional dimension D2.

The illustrated tissue-removing catheter 10 includes an expansion mechanism for use in circumferentially expanding the abrasive burr 14, more particularly, expanding the tubular body 24. In the illustrated embodiment, the catheter 10 includes a balloon 39 (broadly, an expandable member) positioned in the interior of the abrasive burr 14. The balloon 39 is inflated and deflated to control the cross-sectional dimension of the burr 14. Referring to FIG. 4, the balloon 39 is inflated by delivering fluid (e.g., liquid or gas) through a balloon lumen 40 (e.g., a balloon lumen having an annular cross section surrounding the guidewire lumen 20) in the catheter body 12. Referring to FIG. 1, the balloon lumen 40 may be fluidly connected to a fluid inlet port 42 on the handle 16 for delivering fluid from a fluid source (e.g., a syringe). In other embodiments, the balloon 39 may be inflated or deflated by a rotating pressure port (not shown) on the handle 16, and the handle may include the fluid source. As the balloon 39 is inflated, it pushes the struts 26 of the abrasive burr 14 radially outward to expand the circumference of the burr. When the balloon 39 is deflated, the burr 14 contracts toward its initial cross-sectional dimension. The burr 14 is adjustable to different cross-sectional dimensions based on the amount the balloon 39 is inflated. In one example, the balloon 39 rotates with the drive shaft 12 and the burr 14.

The expansion mechanism may be of other types and configurations for expanding the circumference of the abrasive burr 14. For example, in other embodiments the expansion mechanism may not include a balloon. Instead, the expansion mechanism may include a compressible elastomer, or other mechanism for expanding the circumference of the abrasive burr. Other suitable mechanisms are disclosed in co-pending U.S. Application Serial No. 15/189,785, filed June 22, 2016.

The abrasive burr 14 includes an abrasive exterior surface configured to abrade tissue (e.g., plaque). The abrasive exterior surface may be formed by texturing the tubular body 24 and/or the distal head 34. For example, the tubular body 24 and the distal head 24 may be textured using a laser or may be textured in other ways to form an abrasive exterior surface. In another embodiment, the abrasive exterior surface may be formed by applying abrasive particles, such as diamond-coated or silicon carbide particles (e.g., diamond-coated grit or silicon carbide grit), to the tubular body 24 and/or the distal head 34. In these embodiments and other embodiments, the abrasive exterior surface has a suitable roughness to abrade tissue (e.g., plaque) as the abrasive burr 14 is rotated about the axis A.

In the illustrated embodiment, the abrasive exterior surface of the abrasive burr 14 covers a longitudinal portion of each strut 26 extending from adjacent the proximal ends of the struts to adjacent the distal ends of the struts, and covers at least a majority of the distal head 34. At least a majority of the length each of the struts 26 includes the abrasive exterior surface. In particular, in one embodiment about 60% to about 95% of the length of each of the struts 26 includes the abrasive exterior surface, or in another embodiment about 75% to about 90% of the length of each of the struts 26 includes the abrasive exterior surface, or in another embodiment about 80% to about 90% of the length of each of the struts 26 includes the abrasive exterior surface. In some embodiments, the entire lengths of the struts 26 include the abrasive exterior surface.

In the illustrated embodiment, proximal and distal end portions of the struts 26 (i.e., the tubular body 24) adjacent the respective proximal and distal annular hubs 30, 32 are free from the abrasive exterior surface. For example, the proximal and distal end portions of the struts 26 are not textured to form an abrasive surface and do not include abrasive particulate applied to its exterior. It is believed that because the abrasive exterior surface is not on the proximal and distal end portions of the struts 26, the proximal and distal ends of the struts are more flexible and more readily deflected relative to the proximal and distal annular hubs 30, 32 when the balloon is expanded (or other expansion mechanism is activated). Thus, the illustrated embodiment facilitates expansion and/or contraction of the abrasive burr 14.

In the illustrated embodiment, at least portions of the proximal and distal annular hubs 30, 32 are free from the abrasive exterior surface. In particular, at least portions of the proximal and distal annular hubs 30, 32 adjacent the struts 26 are free from the abrasive exterior surface. For example, the portions of the proximal and distal annular hubs 30, 32 not including the abrasive exterior surface are not textured to form an abrasive surface and do not abrasive particles applied to its exterior. The portions of the proximal and distal annular hubs 30, 32 not including the abrasive exterior surface ensure that the junction between the proximal and distal annular hubs and the respective proximal and distal end portions of the struts 26 are free from the abrasive exterior surface so that junctions are more flexible and can readily function as living hinges. In the illustrated example, the entirety of each of the proximal and distal hubs 30, 32 are free from the abrasive exterior surface. In other embodiments, portions of the proximal and distal annular hubs 30, 32 may include the abrasive exterior surface. In still other embodiments, the entirety of each of the proximal and distal annular hubs 30, 32 may include the abrasive exterior surface.

As shown in FIG. 2, the illustrated abrasive burr 14 includes zones extending circumferentially around the abrasive burr and longitudinally along the abrasive burr. Zone 1 consists of the circumferential and longitudinal portion of the tubular body 14 (e.g., the struts 26) that include the exterior abrasive surface, as described above. Zone 2 consists of the entire distal head 34 which includes the exterior abrasive surface. Zone 3 consists of the proximal annular hub 30 and the circumferential and proximal end portion of the tubular body 14 (e.g., the proximal end portions of the struts 26) which are free from the exterior abrasive surface. Zone 4 consists of the distal annular hub 32 and the circumferential and distal end portion of the tubular body 14 (e.g., the distal end portions of the struts 26) which are free from the exterior abrasive surface.

In one example, the exterior abrasive surfaces in Zones 1 and 2 have generally the same roughness. For example, the exterior abrasive surfaces in Zones 1 and 2 may have a roughness of from about 1 Ra (µm) to about 2 Ra (µm), or from about 4 Ra (µm) to about 10 Ra (µm). Where the exterior abrasive surfaces are defined by abrasive particulate applied to the burr, the Zones 1 and 2 may have a grit of from about 400 to about 600, or from about 200 to about 400

In another example, the exterior abrasive surface in Zone 2 may be coarser than the exterior abrasive surface in Zone 1. In this way, the distal head 34 is configured to more aggressively remove tissue compared to the tubular body 24. In one embodiment, the exterior abrasive surface in Zone 1 may be defined by abrasive particulate (e.g., diamond grit) and the exterior abrasive surface in Zone 2 may be defined by surface texturing (e.g., laser machining) of the tubular body 24. In this embodiment, the exterior abrasive surface in Zone 1 may have a roughness less than the roughness of the exterior abrasive surface in Zone 2, whereby the distal head 34 is more abrasive than the tubular body 24 (e.g., the struts 26). In other words, the exterior abrasive surface in Zone 2 may be coarser than the exterior abrasive surface in Zone 1. In one example, the exterior abrasive surface in Zone 1 may have a roughness from about 1 Ra (µm) to about 2 Ra (µm), and the exterior abrasive surface in Zone 2 may have a grit of from about 200 to about 400, or from about 4 Ra (µm) to about 10 Ra (µm).

In one embodiment of a tissue-removing operation, the tissue-removing catheter 10 is advanced in a body lumen over the guide wire 22 to a target site (e.g., a lesion in the body lumen). At the target site, the cross-sectional dimension of the abrasive burr 14 can be expanded initially (e.g., by using the control handle to inflate the balloon 39) or the cross-sectional dimension of the burr can initially remain in its initial, non-expanded configuration. The tissue-removing catheter 10 is activated using the control handle 16, such as by activating a control lever, button, or other device 19 to activate the motor 18. Upon activating the tissue-removing catheter 10, the drive shaft 12 rotates about the axis A, causing rotation of the abrasive burr 14. The burr 14 abrades tissue in the body lumen, thereby, in one example, expanding a diameter of an opening in the lesion in the body lumen. In one example, the distal head 34 engages the tissue to initially bore into the lesion in the body lumen and increase the diameter of the opening in the lesion. Inside the opening in the lesion, the burr 14 continues to rotate and the struts 26 engage and abrade tissue. The burr 14 can be expanded by inflating the balloon 39 for example to further abrade the tissue and increase the diameter of the opening in the lesion.

The tissue-removing catheter 10 may also be used without a guide wire. In one embodiment, the distal head 34 is used to bore through a near total or total occlusion. The tissue-removing catheter 10 may also be used without imparting rotation of the burr 14. In one embodiment, the burr 14 is used to center the tissue-removing catheter 10 in the true lumen (i.e., in the space remaining between occlusions in the body lumen). The burr 14 can be expanded circumferentially to contact the outer limits of the true lumen, thereby centering the tissue-removing catheter 10 in the true lumen. With the catheter 10 centered in the true lumen, a wire (e.g., guide wire 22) can be advanced beyond the distal head 34 to bore through tissue occluding the body lumen. In one embodiment, the burr 14 can be advanced in the initial configuration to a target site, and then expanded circumferentially at the target site to engage the occlusion to cut or tear the occlusion without rotating the tissue-removing head. In one embodiment, the burr 14 can be advanced beyond a target site, then expanded circumferentially and pulled back over the target site to remove tissue. An aspiration catheter (not shown) can be positioned near that tissue-removing head to collect debris removed by the tissue-removing head.

The tissue-removing catheter 10 facilitates creation of a larger lumen diameter. The burr 14 can increase in cross-sectional dimension to continue to enlarge an existing lumen (e.g., by using multiple passes over the same lesion), treat multiple vessels in the same patient, or treat vessels that have slight to moderate aneurysmal pockets. Because the abrasive burr 14 can decrease in cross-sectional dimension after it has been expanded, it may allow a practitioner to retrieve a stuck head or treat disease that is distal to a treatment barrier such as a stent. The variable cross-sectional dimension of the burr 14 reduces the need for using different size heads to treat multiple diameters or vessels, resulting in decreased costs. It also does not rely on centrifugal force (and thus, require a high-speed, well controlled motor) to maintain contact between the burr 14 and the lesion, but rather expands the head to contact the lesion which may result in further cost savings.

When introducing elements of the present invention or the embodiment(s) thereof, the articles "a", "an", "the" and "said" are intended to mean that there are one or more of the elements. The terms "comprising", "including" and "having" are intended to be inclusive and mean that there may be additional elements other than the listed elements.

## Claims

1. A tissue-removing catheter (10) comprising:
a drive shaft (12) having opposite proximal and distal ends and configured for rotation about a longitudinal axis; and
an abrasive burr (14) operatively connected to the distal end of the drive shaft (12) such that rotation of the drive shaft (12) about the longitudinal axis imparts rotation to the abrasive burr (14), the abrasive burr (14) including
circumferentially spaced longitudinal struts (26) defining longitudinal slots (28) between adjacent struts (26),
proximal and distal hubs (30, 32) secured to the respective proximal and distal ends of the longitudinal struts (26), and
an abrasive exterior surface covering an abrasive longitudinal portion of each strut extending from adjacent the proximal ends of the longitudinal struts (26) to adjacent the distal ends of the longitudinal struts (26),
wherein proximal and distal longitudinal end portions of the longitudinal struts (26) adjacent the junctions of the longitudinal struts (26) and the respective proximal and distal hubs (30, 32) are free from the abrasive exterior surface.

2. The tissue-removing catheter (10) set forth in claim 1, wherein at least a distal end portion of the proximal hub (30) adjacent the junctions of the longitudinal struts (26) are free from the abrasive exterior surface, wherein at least a proximal end portion of the distal hub (32) adjacent the junctions of the longitudinal struts (26) are free from the abrasive exterior surface.

3. The tissue-removing catheter (10) set forth in claim 2, wherein an entirety of the proximal hub (30) is free from the abrasive exterior surface, wherein an entirety of the distal hub (32) is free from the abrasive exterior surface.

4. The tissue-removing catheter (10) set forth in claim 3, wherein the abrasive burr (14) further includes a distal head disposed distal of the distal hub (32), wherein the abrasive exterior surface covers at least a portion of the distal head.

5. The tissue-removing catheter (10) set forth in claim 4, wherein the distal head has a cross-sectional dimension tapering distally.

6. The tissue-removing catheter (10) set forth in claim 5, wherein the distal head has a generally conical shape, wherein optionally the abrasive exterior surface covers an entirety of the distal head.

7. The tissue-removing catheter (10) set forth in claim 1, wherein the abrasive burr (14) is selectively adjustable from an initial cross-sectional dimension to an expanded cross-sectional dimension larger than the initial cross-sectional dimension, in which the longitudinal struts (26) are deflected radially outward relative to the proximal and distal hubs (30, 32).

8. The tissue-removing catheter (10) set forth in claim 7, further comprising an expansion mechanism configured to selectively adjust the abrasive burr (14) from the initial cross-sectional dimension to the expanded cross-sectional dimension, wherein optionally the expansion mechanism includes an inflatable balloon (39) disposed inside the abrasive burr (14).

9. The tissue-removing catheter (10) set forth in claim 1, wherein the abrasive exterior surface comprises at least one of textured exterior surfaces of the longitudinal struts, and an abrasive material applied to the exterior surfaces of the longitudinal struts (26).

10. The tissue-removing catheter (10) set forth in claim 9, wherein the abrasive exterior surface comprises the abrasive material, wherein optionally the abrasive material is diamond-coated grit.

11. The tissue-removing catheter (10) set forth in claim 9, wherein the abrasive exterior surface comprises the textured exterior surfaces of the longitudinal struts (26), wherein optionally the textured exterior surfaces of the longitudinal struts (26) are formed by laser machining.

12. The tissue-removing catheter (10) set forth in claim 1, wherein the abrasive burr (14) further includes a distal head disposed distal of the distal hub, wherein the abrasive exterior surface covers at least a portion of the distal head.

13. The tissue-removing catheter (10) set forth in claim 12, wherein the abrasive exterior surface on the distal head has a roughness greater than the roughness of the abrasive exterior surface on the longitudinal struts (26), wherein optionally the abrasive exterior surface on the distal head comprises diamond-coated grit, wherein the abrasive exterior surface on the longitudinal struts (26) comprises textured exterior surfaces of the longitudinal struts (26).

14. A method of forming an abrasive burr (14) for a catheter (10), the method comprising:
forming circumferentially spaced longitudinal struts (26) defining longitudinal slots between adjacent struts (26),
forming proximal and distal hubs (30, 32) secured to the respective proximal and distal ends of the longitudinal struts (26), and
forming an abrasive exterior surface covering an abrasive longitudinal portion of each strut extending from adjacent the proximal ends of the longitudinal struts (26) to adjacent the distal ends of the longitudinal struts (26), wherein proximal and distal longitudinal end portions of the longitudinal struts (26) adjacent the junctions of the longitudinal struts (26) and the respective proximal and distal hubs (30, 32) are free from the abrasive exterior surface, wherein the method further comprising:
forming a distal head connected to and disposed distal of the distal hub; and
forming a second abrasive exterior surface covering at least a portion of the distal head.

## Patentansprüche

1. Gewebeentfernungskatheter (10), der Folgendes umfasst:
eine Antriebswelle (12), die ein proximales und ein distales Ende gegenüberliegend aufweist und für eine Drehung um eine Längsachse konfiguriert ist; und
einen Schleifbohrer (14), der mit dem distalen Ende der Antriebswelle (12) derart wirkverbunden ist, dass die Drehung der Antriebswelle (12) um die Längsachse dem Schleifbohrer (14) Drehung erteilt, wobei der Schleifbohrer (14) Folgendes beinhaltet:
in Umfangsrichtung beabstandete Längsstreben (26), die Längsschlitze (28) zwischen angrenzenden Streben (26) definieren,
eine proximale und eine distale Nabe (30, 32), die an den jeweiligen proximalen und distalen Enden der Längsstreben (26) befestigt sind, und
eine abschleifende Außenoberfläche, die einen abschleifenden Längsabschnitt jeder Strebe bedeckt, der sich von angrenzend an die proximalen Enden der Längsstreben (26) bis angrenzend an die distalen Enden der Längsstreben (26) erstreckt,
wobei proximale und distale Längsendabschnitte der Längsstreben (26), die an die Anschlussstellen der Längsstreben (26) und der jeweiligen proximalen und distalen Nabe (30, 32) angrenzen, von der abschleifenden Außenoberfläche frei sind.

2. Gewebeentfernungskatheter (10) nach Anspruch 1, wobei wenigstens ein distaler Endabschnitt der proximalen Nabe (30), der an die Anschlussstellen der Längsstreben (26) angrenzt, von der abschleifenden Außenoberfläche frei ist, wobei wenigstens ein proximaler Endabschnitt der distalen Nabe (32), der an die Anschlussstellen der Längsstreben (26) angrenzt, von der abschleifenden Außenoberfläche frei ist.

3. Gewebeentfernungskatheter (10) nach Anspruch 2, wobei eine Gesamtheit der proximalen Nabe (30) von der abschleifenden Außenoberfläche frei ist, wobei eine Gesamtheit der distalen Nabe (32) von der abschleifenden Außenoberfläche frei ist.

4. Gewebeentfernungskatheter (10) nach Anspruch 3, wobei der Schleifbohrer (14) ferner einen distalen Kopf beinhaltet, der distal der distalen Nabe (32) angeordnet ist, wobei die abschleifende Außenoberfläche wenigstens einen Abschnitt des distalen Kopfes bedeckt.

5. Gewebeentfernungskatheter (10) nach Anspruch 4, wobei der distale Kopf eine Querschnittsabmessung aufweist, die sich distal verjüngt.

6. Gewebeentfernungskatheter (10) nach Anspruch 5, wobei der distale Kopf eine im Allgemeinen konische Form aufweist, wobei optional die abschleifende Außenoberfläche eine Gesamtheit des distalen Kopfes bedeckt.

7. Gewebeentfernungskatheter (10) nach Anspruch 1, wobei der Schleifbohrer (14) von einer anfänglichen Querschnittsabmessung zu einer erweiterten Querschnittsabmessung, die größer als die anfängliche Querschnittsabmessung ist, in der die Längsstreben (26) relativ zu der proximalen und der distalen Nabe (30, 32) radial nach außen ausgelenkt werden, wahlweise einstellbar ist.

8. Gewebeentfernungskatheter (10) nach Anspruch 7, der ferner einen Erweiterungsmechanismus umfasst, der konfiguriert ist, um den Schleifbohrer (14) von der anfänglichen Querschnittsabmessung zu der erweiterten Querschnittsabmessung wahlweise einzustellen, wobei optional der Erweiterungsmechanismus einen aufblasbaren Ballon (39) beinhaltet, der innerhalb des Schleifbohrers (14) angeordnet ist.

9. Gewebeentfernungskatheter (10) nach Anspruch 1, wobei die abschleifende Außenoberfläche strukturierte Außenoberflächen der Längsstreben und/oder einen Schleifstoff umfasst, der auf die Außenoberflächen der Längsstreben (26) aufgebracht ist.

10. Gewebeentfernungskatheter (10) nach Anspruch 9, wobei die abschleifende Außenoberfläche den Schleifstoff umfasst, wobei optional der Schleifstoff ein diamantbeschichtetes Abreibungsmittel ist.

11. Gewebeentfernungskatheter (10) nach Anspruch 9, wobei die abschleifende Außenoberfläche die strukturierten Außenoberflächen der Längsstreben (26) umfasst, wobei optional die strukturierten Außenoberflächen der Längsstreben (26) durch Laserbearbeitung ausgebildet sind.

12. Gewebeentfernungskatheter (10) nach Anspruch 1, wobei der Schleifbohrer (14) ferner einen distalen Kopf beinhaltet, der distal der distalen Nabe angeordnet ist, wobei die abschleifende Außenoberfläche wenigstens einen Abschnitt des distalen Kopfes bedeckt.

13. Gewebeentfernungskatheter (10) nach Anspruch 12, wobei die abschleifende Außenoberfläche auf dem distalen Kopf eine Rauheit aufweist, die größer als die Rauheit der abschleifenden Außenoberfläche auf den Längsstreben (26) ist, wobei optional die abschleifende Außenoberfläche auf dem distalen Kopf das diamantbeschichtete Abreibungsmittel umfasst, wobei die abschleifende Außenoberfläche auf den Längsstreben (26) strukturierte Außenoberflächen der Längsstreben (26) umfasst.

14. Verfahren zum Ausbilden eines Schleifbohrers (14) für einen Katheter (10), wobei das Verfahren Folgendes umfasst:
Ausbilden von in Umfangsrichtung beabstandeten Längsstreben (26), die Längsschlitze zwischen angrenzenden Streben (26) definieren,
Ausbilden der proximalen und der distalen Nabe (30, 32), die an den jeweiligen proximalen und distalen Enden der Längsstreben (26) befestigt sind, und
Ausbilden einer abschleifenden Außenoberfläche, die einen abschleifenden Längsabschnitt jeder Strebe bedeckt, der sich von angrenzend an die proximalen Enden der Längsstreben (26) bis angrenzend an die distalen Enden der Längsstreben (26) erstreckt, wobei proximale und distale Längsendabschnitte der Längsstreben (26), die an die Anschlussstellen der Längsstreben (26) und der jeweiligen proximalen und distalen Nabe (30, 32) angrenzen, von der abschleifenden Außenoberfläche frei sind, wobei das Verfahren ferner Folgendes umfasst:
Ausbilden eines distalen Kopfes, der mit der distalen Nabe verbunden und von dieser distal angeordnet ist; und
Ausbilden einer zweiten abschleifenden Außenoberfläche, die wenigstens einen Abschnitt des distalen Kopfes bedeckt.

## Revendications

1. Cathéter d'ablation de tissus (10), comprenant :
un arbre d'entraînement (12) comportant des extrémités proximale et distale opposées, et étant conçu pour tourner autour d'un axe longitudinal ; et
une tête de fraisage abrasive (14) reliée fonctionnellement à l'extrémité distale de l'arbre d'entraînement (12) de telle sorte que la rotation de l'arbre d'entraînement (12) autour de l'axe longitudinal transmet une rotation à la tête de fraisage abrasive (14), la tête de fraisage abrasive (14) comprenant
des entretoises (26) longitudinales espacées circonférentiellement, définissant des fentes (28) longitudinales entre des entretoises (26) adjacentes,
des moyeux (30, 32) proximal et distal fixés aux extrémités proximale et distale respectives des entretoises (26) longitudinales, et
une surface extérieure abrasive recouvrant une partie longitudinale abrasive de chaque entretoise s'étendant depuis la partie adjacente aux extrémités proximales des entretoises (26) longitudinales jusqu'à la partie adjacente aux extrémités distales des entretoises (26) longitudinales,
dans lequel les parties d'extrémité longitudinales proximale et distale des entretoises (26) longitudinales adjacentes aux jonctions des entretoises (26) longitudinales et aux moyeux (30, 32) proximal et distal respectifs sont exemptes de la surface extérieure abrasive.

2. Cathéter d'ablation de tissus (10) selon la revendication 1, dans lequel au moins une partie d'extrémité distale du moyeu (30) proximal adjacente aux jonctions des entretoises (26) longitudinales est exempte de la surface extérieure abrasive, dans lequel au moins une partie d'extrémité proximale du moyeu (32) distal adjacente aux jonctions des entretoises (26) longitudinales est exempte de la surface extérieure abrasive.

3. Cathéter d'ablation de tissus (10) selon la revendication 2, dans lequel l'ensemble du moyeu (30) proximal est exempt de la surface extérieure abrasive, dans lequel l'ensemble du moyeu (32) distal est exempt de la surface extérieure abrasive.

4. Cathéter d'ablation de tissus (10) selon la revendication 3, dans lequel la tête de fraisage abrasive (14) comporte en outre une tête distale disposée de manière distale par rapport au moyeu (32) distal, dans lequel la surface extérieure abrasive recouvre au moins une partie de la tête distale.

5. Cathéter d'ablation de tissus (10) selon la revendication 4, dans lequel la tête distale présente une dimension en coupe transversale, laquelle s'effile de manière distale.

6. Cathéter d'ablation de tissus (10) selon la revendication 5, dans lequel la tête distale présente une forme généralement conique, dans lequel la surface extérieure abrasive recouvre éventuellement l'ensemble de la tête distale.

7. Cathéter d'ablation de tissus (10) selon la revendication 1, dans lequel la tête de fraisage abrasive (14) est réglable sélectivement d'une dimension en coupe transversale initiale à une dimension en coupe transversale expansée supérieure à la dimension en coupe transversale initiale, les entretoises (26) longitudinales étant déviées de manière radiale vers l'extérieur par rapport aux moyeux (30, 32) proximal et distal.

8. Cathéter d'ablation de tissus (10) selon la revendication 7, comprenant en outre un mécanisme d'expansion conçu pour ajuster sélectivement la tête de fraisage abrasive (14) de la dimension en coupe transversale initiale à la dimension en coupe transversale expansée, dans lequel éventuellement, le mécanisme d'expansion comporte un ballonnet gonflable (39) disposé à l'intérieur de la tête de fraisage abrasive (14).

9. Cathéter d'ablation de tissus (10) selon la revendication 1, dans lequel la surface extérieure abrasive comprend des surfaces extérieures texturées des entretoises longitudinales et/ou un matériau abrasif appliqué sur les surfaces extérieures des entretoises (26) longitudinales.

10. Cathéter d'ablation de tissus (10) selon la revendication 9, dans lequel la surface extérieure abrasive comprend le matériau abrasif, dans lequel éventuellement, le matériau abrasif est un abrasif revêtu de diamant.

11. Cathéter d'ablation de tissu (10) selon la revendication 9, dans lequel la surface extérieure abrasive comprend les surfaces extérieures texturées des entretoises (26) longitudinales, dans lequel les surfaces extérieures texturées des entretoises (26) longitudinales sont éventuellement formées par usinage laser.

12. Cathéter d'ablation de tissus (10) selon la revendication 1, dans lequel la tête de fraisage abrasive (14) comporte en outre une tête distale disposée de manière distale par rapport au moyeu distal, dans lequel la surface extérieure abrasive recouvre au moins une partie de la tête distale.

13. Cathéter d'ablation de tissus (10) selon la revendication 12, dans lequel la surface extérieure abrasive sur la tête distale présente une rugosité supérieure à la rugosité de la surface extérieure abrasive sur les entretoises (26) longitudinales, dans lequel éventuellement, la surface extérieure abrasive sur la tête distale comprend de l'abrasif revêtu de diamant, dans lequel la surface extérieure abrasive sur les entretoises (26) longitudinales comprend des surfaces extérieures texturées des entretoises (26) longitudinales.

14. Procédé de formation d'une tête de fraisage abrasive (14) pour un cathéter (10), ledit procédé comprenant :
la formation des entretoises (26) longitudinales espacées circonférentiellement, définissant des fentes longitudinales entre des entretoises (26) adjacentes,
la formation des moyeux (30, 32) proximal et distal fixés aux extrémités proximale et distale respectives des entretoises (26) longitudinales, et
la formation d'une surface extérieure abrasive recouvrant une partie longitudinale abrasive de chaque entretoise s'étendant depuis la partie adjacente aux extrémités proximales des entretoises (26) longitudinales jusqu'à la partie adjacente aux extrémités distales des entretoises (26) longitudinales, dans lequel les parties d'extrémité longitudinales proximale et distale des entretoises (26) longitudinales adjacentes aux jonctions des entretoises (26) longitudinales et aux moyeux (30, 32) proximal et distal respectifs sont exemptes de la surface extérieure abrasive, dans lequel ledit procédé comprenant en outre :
la formation d'une tête distale reliée au moyeu distal et disposée de manière distale par rapport à celui-ci ; et
la formation d'une seconde surface extérieure abrasive recouvrant au moins une partie de la tête distale.
